# EUROPEAN PATENT SPECIFICATION

(11) **EP 3 344 298 B1**
(45) Date of publication and mention of the grant of the patent: **11.02.2026**
(21) Application number: 16741741.9
(22) Date of filing: 01.07.2016
(51) Int. Cl.: A61K 51/12, A61P 35/00, A61B 18/00, A61N 5/10

(54) **SYSTEM FOR RADIATION THERAPY**
SYSTEM FÜR STRAHLENTHERAPIE
SYSTÈME DE RADIOTHÉRAPIE

(30) Priority: 06.09.2015 US 201514846817; 30.06.2016 US 201615197764
(43) Date of publication of application: 11.07.2018
(73) Proprietor: Sirtex Medical Inc., Woburn, Massachusetts 01887 (US)
(72) Inventor: SRINIVAS, Shyam, Cupertino, CA 95014 (US); DOKE, Aniruddha, Highland Heights, OH 44143 (US)
(74) Representative: Kehoe, Laura Ellen
(86) International application number: PCT/US2016/040599
(87) International publication number: WO 2017/039822

(56) References cited:
- WO-A1-02/34300
- WO-A1-2014/040143
- WO-A2-2005/061009
- MIEDERER M ET AL: "Realizing the potential of the Actinium-225 radionuclide generator in targeted alpha particle therapy applications", ADVANCED DRUG DELIVERY REVIEWS, ELSEVIER, AMSTERDAM, NL, vol. 60, no. 12, 15 September 2008 (2008-09-15), pages 1371 - 1382, XP022851265, ISSN: 0169-409X, [retrieved on 20080423], DOI: 10.1016/J.ADDR.2008.04.009
- MAARTEN AD VENTE ET AL: "Microspheres for radioembolization of liver malignancies", EXPERT REVIEW OF MEDICAL DEVICES, vol. 7, no. 5, 1 September 2010 (2010-09-01), GB, pages 581 - 583, XP055305366, ISSN: 1743-4440, DOI: 10.1586/erd.10.48
- RAYMOND J ET AL: "Production of radioactive particles for endovascular therapeutic interventions", BIOMATERIALS, ELSEVIER SCIENCE PUBLISHERS BV., BARKING, GB, vol. 27, no. 8, 1 March 2006 (2006-03-01), pages 1566 - 1572, XP027950912, ISSN: 0142-9612, [retrieved on 20060301]

## Description

### TECHNICAL FIELD

This invention relates to a radiomicrosphere for use in the treatment of cancer.

### BACKGROUND ART

Hepatocellular carcinoma ("HCC") is the third leading cause of cancer related deaths and the sixth most prevalent cancer as ~750,000 new cases are diagnosed each year that result in ~700,000 deaths worldwide. Axelrod and von Leeuwen have reported that the incidence of HCC has "more than doubled, from 2.6 to 5.2 per 100,000 population" over the past 20 years, with an increase in mortality from 2.8 to 4.7 per 100,000. 80% of the HCC cases are due to the early acquisition of hepatitis B and C in conjunction with high-risk behavior. Additionally, the obesity epidemic has contributed to an increase in non-alcoholic steatohepatitis (NASH), which can eventually progress to fibrosis, cirrhosis, and HCC.

The treatment of HCC has been challenging, since most patients present at an advanced stage. Symptoms of liver cancer are often vague and don't appear until the cancer is at an advanced stage. In early stages of the disease, surgical treatments like resection and transplantation provide the best curative outcomes. A disadvantage of resection, however, is that patients' remnant livers may not be able to support the necessary hepatic functional demands, and there is a high potential for recurrent disease. Moreover, there are ~35,000 patients diagnosed with HCC annually in the US alone of which ~80% have disseminated unresectable tumors. Other treatment modalities include transarterial chemoembolization (TACE), sorafenib chemotherapy, external beam radiation, and radiofrequency ablation. In comparison to sorafenib and external beam radiation, more local therapies such as radiofrequency ablation, radioembolization (RE), and TACE are able to deliver the desired dose to the target with minimal toxicity to the system. Supporting this statement, Dezarn et al. noted that the maximum external beam acceptable dose to the whole liver of 35 Gy delivered in 1.8 Gy/day fractions is far below the 70 Gy typically needed to destroy solid tumor lesions. The high sensitivity of normal hepatic tissue to external beam radiation has given way to more locally effective RE or selective internal radiation therapy (SIRT) techniques.

RE, a promising catheter based liver-directed modality indicated for HCC, is the transcatheter angiographic delivery of microspheres. Injection of the microspheres via the hepatic artery provides advantage as observations have demonstrated that metastatic hepatic malignancies > 3mm derive ~80-100% of their blood supply from the arterial rather than the portal hepatic circulation. The normal liver tissue is predominantly fed by the portal vein (60-70%). Current Yttrium-90 (⁹⁰Y) spheres trapped at the precapillary level emit internal β radiation, providing a relatively more localized higher dose delivery as compared to external beam radiation. However, the average energy of 0.94 MeV of ⁹⁰Y β emission that delivers ~49.38Gy/kg/GBq to tissue, and its longer path length (mean tissue penetration of 2.5 mm and a maximum range of 1.1 cm) result in collateral damage of the normal liver. Radiation damage due to the longer path length is amplified as ~25% of liver is also fed by the hepatic artery. Thus, normal liver tissue can receive doses that are non-trivial with ensuing side effects. Side effects from β radiation to the normal liver can cause nausea, discomfort and liver dysfunction. Abnormal high radiation doses to the normal tissue may even result in radiation-induced hepatitis with potential risk of liver failure.

The other disadvantage of prior art ⁹⁰Y microspheres is their inability to be imaged easily with high quality and quickly acquired images on par with other currently used diagnostic isotopes. Dosimetry in ⁹⁰Y radioembolization is largely empirical when following current manufacturer recommended guidelines of both glass and resin microsphere products. Little is known about the actual radiation absorbed dose to both tumor and normal liver in these treatments. Thus, the prior art dosing regimen is suboptimal for tumor kill, sparing of normal liver, and does not take advantage of the newer techniques of quantitative Positron Emission Tomography/ Computerized Tomography (PET/CT) for accurate dosimetry. WO2005/061009 A2 relates to microparticles for microarterial imaging and radiotherapy, the microparticles comprising a core, at least one linking carrier on the core, and at least one radioactive therapeutic agent covalently bonded to the linking carrier.

### SUMMARY OF THE INVENTION

The present invention relates to a radiomicrosphere for use in the treatment of cancer with radioembolization as defined in the claims. The targeted organ may be the liver and the disease state that is being treated may be hepatocellular carcinoma (HCC). The radiomicrosphere is formed from a resin where at least one isotope attached to the resin is for tumoricidal therapy. The at least one isotope emits primarily alpha particles and as the at least one isotope decays, daughter radionuclide of the alpha decay are captured by the resin. The resin is a cation exchange resin and is polyfunctional where the resin has at least three different types of functional groups for cation binding. The at least three functional groups bonded to the resin include a carboxylic acid group, a diphosphonic acid group, and a sulfonic acid group. In embodiments, the device further comprises a second isotope for dosimetric purposes, where the second isotope is a positron emitter for PET based dosimetry. In specific embodiments, the first isotope is Actinium-225 (²²⁵Ac), and the second isotope is Zirconium-89 (⁸⁹Zr). The radiomicrosphere may have at least 5 times greater tumoricidal efficacy than existing 90Y radioembolization techniques. In further embodiments of the present invention, an amount of radiation dose absorbed to both tumor cells and normal liver cells after radioembolization can be determined within 5 minutes of the start of the PET scan. In preferred embodiments, each radiation treatment contains a total number of around 37 million radiomicrospheres. In still further embodiments, the radiomicrosphere consists of a cation exchange resin with at least one radionuclide therein. In embodiments, the daughter radionuclides of the at least one isotope decay are captured at high efficiency near 100%. In embodiments, the radiomicrosphere is implanted in a targeted treatment area.

Other features and advantages of the invention will become apparent from the following detailed description, taken in conjunction with the accompanying drawings that illustrate, by way of example, various features of embodiments of the invention.

### BRIEF DESCRIPTION OF THE DRAWINGS

A detailed description of embodiments of the invention will be made with reference to the accompanying drawings, wherein like numerals designate corresponding parts in the several figures.
FIG. 1 is a schematic view of the radiomicrosphere used for RE in accordance with a preferred embodiment of the present invention; and
FIG. 2 is an illustrative cross sectional view of a liver sinusoid with use of the radiomicrosphere in FIG. 1 in accordance with a preferred embodiment of the present invention.

### DESCRIPTION OF EMBODIMENTS

As shown in the drawings for purposes of illustration, the invention is embodied in a new radiomicrosphere for use in the treatment of cancer with radioembolization. The new radiomicrosphere can significantly extend the overall survival for patients with primary liver cancer also known as hepatocellular carcinoma (HCC), while providing distinct advantages and features over the prior art. However, it will be recognized that further embodiments of the invention may be used for other disease states including cancer in other parts of the body.

The current prior art when treating HCC using radioembolization (RE) is the transcatheter angiographic delivery of Yttrium-90 (⁹⁰Y) microspheres. The disadvantages with ⁹⁰Y RE are the high beta radiation to the normal liver (collateral damage), suboptimal dosing regimens for tumor kill and the lack of quantitative imaging for accurate dosimetry. The preferred embodiments of the present invention replaces ⁹⁰Y microspheres with a new theranostic (i.e. therapeutic + diagnostic) agent designed to be the next generation of radiomicrosphere.

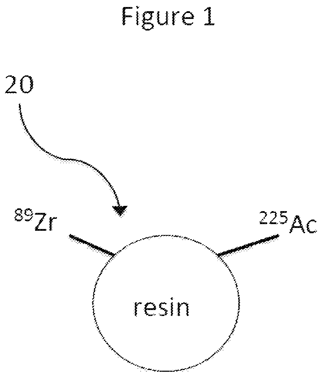

FIG. 1 illustrates the radiomicrosphere 20 in accordance with the preferred embodiments of the present invention. Radiomicrosphere 20 is the first theranostic sphere in commonly used RE products. In preferred embodiments, radiomicrosphere 20 is a resin microsphere with both a diagnostic isotope, Zirconium-89 (⁸⁹Zr), a positron emitter for PET based dosimetry, and a therapeutic isotope, Actinium-225 (²²⁵Ac), an alpha emitter for therapy. The innovation lies in the dual nature of the agent that offers both focal therapy and quantitative dosimetry simultaneously for the treatment f HCC.

The advantage of radiomicrospheres 20 over existing ⁹⁰Y radiomicrospheres is the greater tumoricidal effect with a lesser amount of radiation (and thus less collateral damage) as well as provide valuable dosimetry and diagnostic information. On the therapeutic aspect, radiomicrospheres 20 is advantageous over existing ⁹⁰Y radiomicrospheres as the ²²⁵Ac alpha particle will have greater tumoricidal efficacy with a lesser amount of radiation due to the 5x-10x greater biological effect from alpha particles vs. ⁹⁰Y beta particles. The lethal dose is delivered by the four alpha particles in the decay chain of ²²⁵Ac. Alpha particles result in a greater number of double stranded DNA breaks, which are lethal events for cells as opposed to a single stranded DNA break from a beta particle that is more easily repairable. In addition, alpha particles result in less "collateral damage" due to their greater mass and shorter path length in tissue. The estimated path length of ²²⁵Ac alpha particle is 80-100 µm where a ⁹⁰Y beta particle is on the order of 1-2.5 mm. Also, the two betas from ²²⁵Ac decay have much lower energy (444 keV, 659 keV and 198 keV) and mean path lengths (first β 98% chance of 1.2mm; 2% chance of 1.8mm; second β 100% chance of 0.5mm). A good example of fewer side effects from alpha emitter therapy when compared to beta emitters, is the current clinical use of ²²³Ra, the first FDA approved alpha emitter for the treatment of osseous metastases in prostate cancer. ²²³Ra has a much kinder side effect profile with less bone marrow suppression than its beta emitter predecessors namely ¹⁵³Sm and ⁸⁹Sr.

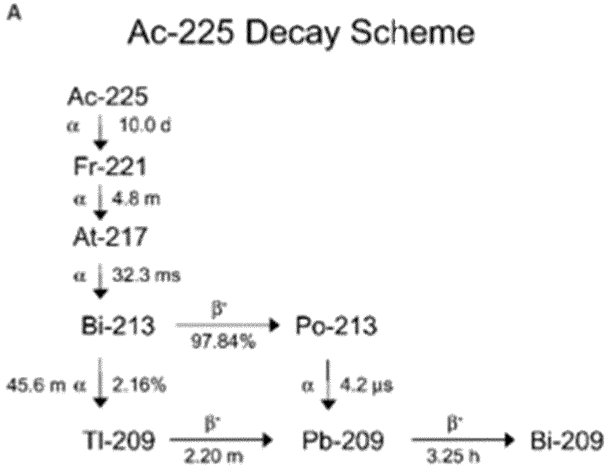

One difficulty well known in the field of alpha particle therapy has been the containment of daughter radionuclide. The alpha particle has the mass of a helium nucleus and therefore has a lot of momentum associated with its ejection from a radioactive nucleus. This momentum results in a strong "alpha recoil" which is a consequence of the alpha particle decay. The net result is that a daughter radionuclide which results from the alpha decay can break free from its chelator, peptide, antibody, or whatever ligand to which it is attached. The daughter radionuclides can then freely float in the circulation and cause unwanted side effects. In the case of Actinium-225 decay, these daughters are ²²¹Fr (t_{1/2} = 4.8 m; 6 MeV α particle and 218 keV γ emission), ²¹⁷At (t_{1/2} = 32.3 ms; 7 MeV α particle), ²¹³Bi (t_{1/2} = 45.6 m; 6 MeV α particle, 444 keV β- particle and 440 keV γ emission), ²¹³Po (t_{1/2} = 4.2 µs; 8 MeV α particle), ²⁰⁹Tl (t_{1/2} = 2.2 m; 659 keVβ-particle), ²⁰⁹Pb (t_{1/2} = 3.25 h; 198 keV β- particle) and ²⁰⁹Bi (stable isotope, no decay). The most well known side effect is Bismuth-213 causing renal toxicity. In mice, renal irradiation from free, radioactive daughters of ²²⁵Ac, led to time-dependent reduction in renal function manifesting as pallor and an increase in blood urea nitrogen. Corresponding histopathological changes were observed in the kidneys. Glomerular and tubular cell nuclear pleomorphism, karyorrhexis, tubular cell injury and lysis were observed as early as 10 weeks. Progressive thinning of the cortex due to widespread tubulolysis, collapsed tubules, glomerular crowding, decrease in glomerular cellularity and interstitial inflammation and an elevated juxtaglomerular index were noted at 20 - 30 weeks post treatment. By 35 - 40 weeks, regeneration of simplified tubules with tubular atrophy and loss and focal interstitial fibrosis had occurred. A lower juxtaglomerular cell index with focal cytoplasmic vacuolization, suggesting increased degranulation, was also observed in this period. Increased tubular and interstitial TGF-β₁ expression and a corresponding increase in the extracellular matrix deposition was noticed only at 40 weeks post injection. These findings suggest that internally delivered alpha particle radiation-induced loss of tubular epithelial cells triggers a chain of adaptive changes that result in progressive morphological damage accompanied by a loss of renal function [ref: Miederer M, Scheinberg D, and McDevitt M. Adv Drug Deliv Rev. 2008 Sep; 60(12): 1371-1382.].

In accordance with the present invention, the choice of a resin used in radiomicrosphere 20 is based on the ability to control the free floating daughters produced by Alpha emitters. We have employed a cation exchange resin currently utilized in the removal of metals from wastewater, actinide separation procedures, and in treatment of radioactive waste from nuclear power and fuel processing plants. The resin is constructed of a polystyrene/divinylbenzene matrix in a spherical bead form. The resin is polyfunctional, having 3 different types of functional groups for cation binding. These are namely a carboxylic acid group, a diphosphonic acid group, and a sulfonic acid group bonded to the polymer matrix. The diphosphonic acid ligands contribute to the resin's unique selective capability by preferentially removing selected metals from the solution. The hydrophilic sulfonic acid ligands enhance metal ion accessibility into the polymer matrix and significantly improve the exchange kinetics. This resin's ability to remove up to 2 Ci from a liter of solution makes it an ideal candidate for binding Actinium-225 and its daughters. Based on the selection of this particular resin, the capture and containment of Ac-225's daughters are near 100%. The resin may contain different polymer backbone as well as different functional groups that provide similar advantages and results. For example, other functional groups with specific metal trapping and limited usage range may include one or more of the following: thiol, aminophosphonic acid, iminodiacetic acid, and bis-picolyl amine (for metals at pH2 to 5). However, the method for controlling the detachment of daughter radionuclide resulting from alpha decay using the resin itself is novel and unique and represents a technique that is unprecedented. It solves the long-standing and complex problem of free daughter radionuclides from alpha decay that may disperse in the media. The embodiments of the present invention provide a solution giving utility to this particular resin while being able to support a dual isotope radiomicrosphere device.

According to another preferred embodiment of the present invention, a uniqueness of this radiomicrosphere 20 construct is to have diagnostic dosimetric information obtained simultaneously once the therapy has been deployed. The quantitative power of PET can be utilized to determine the radiation absorbed dose to both tumor and normal liver. Traditionally in ⁹⁰Y RE, post therapy imaging is not part of the treatment paradigm in a clinical setting. Although positron emission does occur in ⁹⁰Y, its positron branching fraction is extremely small (32ppm), making it a non-ideal imaging agent and also requires patients to lay still in a PET/CT scanner for 20-30 minutes for image acquisition. Contrast that with ⁸⁹Zr which has a 22.6% positron branching fraction, making it an ideal diagnostic agent. A PET/CT scan from ⁸⁹Zr can be collected in a 3-7 minute time span and their quality will be far superior because of the higher positron count rate. The concurrent PET scan of the treatment would offer valuable information to understanding the efficacy, dosage of the treatment, and also the potential damage to the normal liver. This quantitative information can guide the future management of the patient, alerting clinicians when follow up treatments or closer surveillance of liver function may be necessary. Furthermore, the quantitative power of PET based dosimetry can show whether tumors respond to such radiation treatment. It has been discovered that even after receiving what would be considered tumoricidal doses of radiation, many tumors do not respond. The dosimetry method proposed here is also novel. The alpha particle radiation absorbed dose can be calculated from models that have been developed after the distribution of the alpha emitters is known. Since the Zr-89 will be co-labeled on the same radiomicrosphere as Ac-225, it allows us to use the Zr-89 distribution as a surrogate for the Ac-225 distribution. Furthermore, since the Zr-89 and Ac-225 will have a predefined ratio of activity on each microsphere, being able to measure the counts on a ⁸⁹Zr PET/CT scan will readily allow conversion to a number of Ac-225 counts. The quantitative information for Ac-225 will then come from converting the activity in Bq to the actual radiation absorbed dose in Gy within a defined volume of interest. This proposed method of using a positron emitter (Zr-89) as a surrogate for quantity and distribution of alpha emitter (Ac-225) is a unique and novel method of dosimetry. A local deposition method using MIRD dosimetry or a convolution kernel can be employed (both accepted methods) for converting activity to radiation absorbed dose. This scan will give the macroscopic information about dose to tumors and to normal liver tissue.

Positron Emission Tomography (PET) is a powerful technique. It provides two types of information: 1) the reconstructed PET image can tell you whether the positron emitting isotope is present or absent in a particular location. The image it provides is a distribution map of the presence of the isotope being imaged. All different imaging modalities depend on their ability to create contrast. The image created then has a pattern based on its technique which physicians can recognize and use to make a diagnosis. 2) The second type of information is unique to the PET technique in that it is semi-quantitative. There is a number that can be obtained from the information, which can be used for other purposes. In the case of PET, this number refers to the number of radioactive counts within a defined volume of interest (VOI). Again that number can then be exploited for various uses. In the preferred embodiment of Zr-89 attached to a microsphere, we propose the ability to obtain dosimetric information about the quantity of radiomicrosphere present, and in turn calculate a radiation absorbed dose measured in gray (Gy). Since the Zr-89 and Ac-225 will be co-located on a single radiomicrosphere, the presence of radioactive counts from Zr-89 can be converted into radiation absorbed dose in Gy, using the appropriate mathematical conversion (i.e. local deposition method). Therefore in our construct the positron emitter is not for the purpose of imaging, but instead it is for the purpose of dosimetry. The presence or absence of the positron emitter on an image is not the desired technique, but instead the quantity of that positron emitter gives rise to dosimetric information. Imaging and dosimetry are separate and independent goals and are not synonymous with one another by any means.

In alternative embodiments of the present invention, alternative isotopes may be substituted. Examples of alternative isotopes include:
Alpha emitters:
   Terbium-149 (Tb-149)
   Astatine-211 (At-211)
   Bismuth-212 (Bi-212)
   Bismuth-213 (Bi-213)
   Radium-223 (Ra-223)
   Radium-224 (Ra-224)
   Thorium-227 (Th-227)
   Thorium-228 (Th-228)
   Fermium-255 (Fm-255)
Positron emitters:
   Fluorine-18 (F-18)
   Scandium-44 (Sc-44)
   Copper-64 (Cu-64)
   Gallium-68 (Ga-68)
   Yttrium-86 (Y-86)
   Iodine-124 (I-124)
   Terbium-152 (Tb-152)

The radiomicrosphere 20 can be labeled with just an alpha emitter or can be labeled with just a positron emitter. In such cases, a mixture of the microspheres labeled with the alpha emitter and microspheres labeled with the positron emitter may be able to provide similar results as dual labeled microsphere 20 with both an alpha emitter and a positron emitter. In other words, a population of alpha emitter labeled resin microspheres is mixed with the second population of positron emitter labeled resin microspheres prior to implantation in the targeted treatment area. Alternatively, the population of alpha emitter labeled resin microspheres can be implanted in the same targeted treatment area as the population of positron emitter labeled resin microspheres contemporaneously without prior mixing. Certain applications may only require a microsphere labeled with an alpha emitter or a microsphere labeled with a positron emitter. The microsphere with an alpha emitter may be co-labeled with a different isotope for a different imaging or dosimetric purpose or the microsphere with a positron emitter may be co-labeled with a beta emitter.

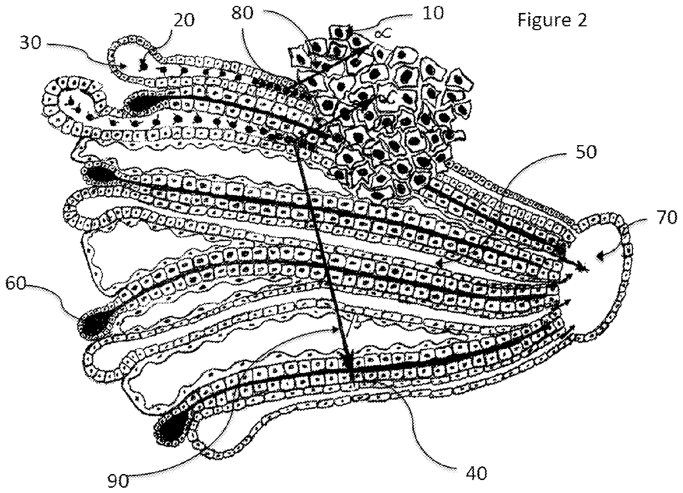

FIG. 2 is an illustrative cross sectional view (highly magnified, not to scale) of a liver sinusoid with use of the radiomicrosphere 20 in FIG. 1. As per RE techniques, the radiomicrospheres 20 are injected intra-arterially into the hepatic arteriole 30 near the targeted area of the liver to treat the hepatocellular carcinoma (HCC) 10. A more detailed description of the RE procedure will be described below. For reference purposes, FIG. 2 is labeled with parts of the liver including normal hepatocytes 40, portal venule 50, bile canaliculi 60, and central vein 70. As seen in FIG. 2, the alpha particle pathway 80 is shown to illustrate the mean free path which is more localized and focused around the HCC tumor 10. Also, the beta particle pathway 90 is shown to illustrate the longer mean free path the beta particle will have in the liver.

The typical sequence of events for a liver cancer patient is as follows: The patient will usually exhibit symptoms or laboratory abnormalities suspicious for a liver tumor. The patient will have CT or MRI imaging to document the presence of a lesion. That lesion will be biopsied to confirm a cancer. Depending on the stage of the cancer, surgery may not be an option (roughly 80% of the time). The patient will be referred to an interventional radiologist for radioembolization treatment. Further specialized imaging may be obtained (i.e. PET/CT, octreotide SPECT, triphasic liver CT or MRI) to better characterize the extent of the tumor. The interventional radiologist will then conduct an anatomic mapping of the patient's liver vasculature. This is essentially an exploration of the vasculature within the liver and discovering which vessel(s) are the optimal route of approach for delivery of the radiomicrospheres 20.

Once the interventional radiologist has placed a catheter in the location which he believes is optimal for delivery, a simulation is performed by injecting technetium-99m tagged to macroaggregated albumin (Tc^{99m}-MAA) as a precursor to the intended RE therapy. The advantage of this agent is that the MAA has a similar size (~30 micron diameter) when compared to the radiomicrospheres 20, and thus the MAA distribution is a good simulation for the eventual distribution of the injected radiomicrospheres 20. The MAA being tagged with Tc^{99m} is readily amenable to SPECT/CT imaging, taking advantage of the anatomical landmarks that come with hybrid imaging. The MAA (being a protein) is digested by enzymes, and therefore its embolization of the tumor vasculature is only transient enough for the imaging. Once images are acquired the MAA is degraded making way for the radiomicrospheres 20 that are to follow in another angiographic session. On occasion the MAA scan may reveal some shunting of blood to other normal organs from the liver vessels. This can manifest itself as uptake in the stomach, duodenum, or sometimes as excessive lung shunting. Such undesirable uptake usually can be prevented with coil embolization to cut off those vascular pathways prior to the RE treatment. In the case of excessive lung shunting, the radiomicrospheres 20 dose would be reduced.

When the patient returns on the actual therapy day, an authorized user of radioactivity (either a radiation oncologist, qualified interventional radiologist, or a nuclear medicine physician) will have calculated the appropriate dose for the patient and will administer this to the patient through the interventional radiologist's catheter after it is placed in the same position as was performed during the MAA simulation. The patient will have confirmatory post-therapy scan (PET/CT) to confirm the distribution of the radiomicrospheres 20 immediately after the therapy and obtain dosimetry information. Follow up of the patient is usually performed by monitoring the patient for any symptoms and by conventional imaging approximately 3 months after the treatment.

Thus, the improved radioembolization techniques provides an invaluable approach to extend the overall survival of patients who otherwise have few options and keep an acceptable quality of life with fewer side effects that are commonly encountered with conventional chemotherapy and current methods of RE.

### Fabrication of the Radiomicrosphere

According to preferred embodiments of the present invention, fabrication of the radiomicrosphere consists of ²²⁵Ac (alpha emitter) and ⁸⁹Zr (positron emitter) both bound to a resin microsphere and verification of its stability at physiologic pH and temperature. The acceptance criteria will be a 97% or greater binding efficiency of isotopes to the resin (i.e., <3% free isotope).

In preferred embodiments of the present invention, ²²⁵Ac and ⁸⁹Zr have been chosen since they have independently been used in human subjects (in various forms, as a single isotope labeled product). The total number of particles will be designed to be 37 million (± 10%,) in each dose. This number was chosen as it makes the dose more embolic than the current commercially available glass microspheres and less embolic than the current commercially available resin microspheres, thought by experts to be respective shortcomings with both products. Based on the various studies with ²²⁵Ac labeled antibodies, we will aim for the following specific activities 100 mBq ²²⁵Ac & 10 Bq ⁸⁹Zr per sphere = total 100 µCi ²²⁵Ac & 10 mCi ⁸⁹Zr.

The basic structure of a radiomicrosphere has a radioisotope element which is bound to a microsphere, usually either glass or resin, with other substrates also being possible. The following four preparation procedures are employed and proposed for the synthesis of our resin based radiomicrospheres 20. The first details a radiomicrosphere consisting of Ac-225 alpha emitting isotope and resin microsphere. The second details a radiomicrosphere consisting of Zr-89 positron emitting isotope and resin microsphere. The third details a radiomicrosphere with dual isotopes Ac-225 and Zr-89 both labeled to a resin microsphere. According to preferred embodiments of the present invention, this co-labeling method with both isotopes on the same resin microsphere is the preferred configuration. The fourth details a mixture of two populations of radiomicrospheres, namely resin microspheres approximately half labeled with Ac-225 and half labeled with Zr-89.

### Example 1: Procedure for Ac-225 labeled microsphere synthesis

Materials and chemicals:
1) ²²⁵Ac (100 µCiin 0.01M HCl, approx. volume = 50-60 µl)
2) Cation exchange resin (~30 micron diam), 0.01M HCl solution
3) Glassware, magnetic stirrer, labware, sterile water for injection (SWFI), sterile pH 7.4 buffer solution (preferably no phosphate)
4) Suitably equipped lab for radiation protection.
5) Dose calibrator and Ge gamma spectroscopy system

Procedure:
1) 0.5 gram of cation exchange resin (~30 micron diam), are added 0.01M HCl solution (5 mL at the beginning and add in increments of 1 mL if needed) in suitable container and stirred using magnetic stir bar to form a reasonable slurry suspension for 30 minutes to ensure complete wetting of the resin.
2) Actinium-225 solution (activity = 100 µCi, in 0.01M HCl, approx. volume 50-60 µl) is added to the flask. Check the pH of the solution (expected to be about pH 2.0) and stirring is resumed for 90 minutes to ensure homogenous distribution of the radioactivity in microspheres.
3) Check for free Ac-225 after centrifugation of the sample by removing a 100 uL aliquot.
4) These labeled microspheres are then filtered using a suitable filtration setup (i.e. 22 micron filter) and washed with 5 ml x 6 times with SWFI. Store each wash separately for alpha counting. Check pH of the last wash solution (expected to be between 6.5-7.0)
5) Resuspend the spheres in sterile pH 7.4 buffer solution (5 mL) to make a suspension. Transfer it to a suitable crimp capped vial. Measure the radioactivity of the microspheres to determine the amount labeled using suitable method.
6) Perform a leaching test for Actinium as follows: The above vial containing microspheres is agitated (shake table) for 20 minutes in a water bath at 37°C. After agitation the vial should remain unagitated at 37°C. After centrifugation, a 100 µL sample is taken from the vial and Ac activity of the supernatant is measured and recorded. Ac activity of the resin is also measured and recorded. Samples should be kept at 37°C during the duration of the leaching test. Perform this test on Day 1, 2, and 3. If the results are acceptable, measure leaching activity at Day 7, 10, and 20.

### Example 2: Procedure for Zr-89 labeled microsphere synthesis

Materials and chemicals:
1) ⁸⁹Zr (approx. 10 mCi in pH 8 buffer)
2) Cation exchange resin (~30 micron diam), pH 8 buffer solution (preferably no phosphate), sterile pH 7.4 buffer solution
3) Glassware, magnetic stirrer, labware, sterile water for injection (SWFI)
4) Suitably equipped lab for radiation protection.
5) Dose calibrator and Ge gamma spectroscopy system

Procedure:
1) 0.5 gram of cation exchange resin (~30 micron diam), are added to pH 8 buffer (5 mL at the beginning and add in increments of 1 mL if needed) in suitable container and stirred using magnetic stir bar to form a reasonable slurry suspension for 30 minutes to ensure complete wetting of the resin.
2) Add the Zr-89 solution in pH 8 buffer to the suspension and continue stirring for 20 minutes.
3) The Zr-89 labeling efficiency is expected to be about 70 % with this procedure.
4) These labeled microspheres are then filtered using a suitable filtration setup (i.e. 22 micron filter) and washed with 5 ml x 6 times with SWFI. Collect all the washes in separate containers to measure labeling efficacy for each isotope.
5) Collect the dual labeled microspheres and resuspend in a vial with 5 ml sterile pH 7.4 buffer solution.
6) Perform leaching tests for Zr-89 at defined time intervals as described in leaching test for Ac-225.

### Example 3: Procedure for Ac-225 and Zr-89 dual labeled microsphere synthesis

Materials and chemicals:
1) ²²⁵Ac (100 µCiin 0.01M HCl, approx. volume = 50-60 µl), ⁸⁹Zr (10 mCi in sterile pH 7.4 buffer)
2) Cation exchange resin (~30 micron diam), 0.01M HCl solution, sterile pH 7.4 buffer solution (preferably no phosphate)
3) Glassware, magnetic stirrer, labware, Sterile water for injection (SWFI)
4) Suitably equipped lab for radiation protection.
5) Dose calibrator and Ge gamma spectroscopy system

Procedure:
1) 0.5 gram of cation exchange resin (~30 micron diam), are added 0.01M HCl solution (5 mL at the beginning and add in increments of 1 mL if needed) in suitable container and stirred using magnetic stir bar to form a reasonable slurry suspension for 30 minutes to ensure complete wetting of the resin.
2) Actinium-225 solution (activity = 100 µCi, in 0.01M HCl, approx. volume 50-60 µl) is added to the flask. Check the pH of the solution (expected to be about pH 2.0) and stirring is resumed for 90 minutes to ensure homogenous distribution of the radioactivity in microspheres.
3) Check for free Ac-225 after centrifugation of the sample by removing a 100 uL aliquot.
4) These labeled microspheres are then filtered using a suitable filtration setup (i.e. 22 micron filter) and washed with 5 ml x 6 times with SWFI.
5) Resuspend these Ac-225 labeled spheres in sterile pH 7.4 buffer in a suitable container and stir using small magnetic stir bar to form a reasonable slurry suspension for 15 minutes.
6) Add the Zr-89 solution to the suspension and continue stirring for 20 minutes.
7) The Zr-89 labeling efficiency is expected to be about 60 % with this procedure.
8) These labeled microspheres are then filtered using a suitable filtration setup (i.e. 22 micron filter) and washed with 5 ml x 6 times with sterile pH 7.4 buffer solution. Collect all the washes in separate containers to measure labeling efficacy for each isotope.
9) Collect the dual labeled microspheres and resuspend in a vial with 5 ml sterile pH 7.4 buffer solution. Perform leaching tests for both radioisotopes at defined time intervals.

### Example 4: Procedure for preparing a mixture of Ac-225 labeled and Zr-89 labeled microspheres

### Part 1) Making Ac-225 labeled microspheres with 2x specific activity

Materials and chemicals:
1) ²²⁵Ac (200 µCi in 0.01M HCl, approx. volume = 60 µl)
2) Cation exchange resin (~30 micron diam), 0.01M HCl solution
3) Glassware, magnetic stirrer, labware, sterile water for injection (SWFI), sterile pH 7.4 buffer solution (preferably no phosphate)
4) Suitably equipped lab for radiation protection.
5) Dose calibrator and Ge gamma spectroscopy system

Procedure:
1) 0.5 gram of cation exchange resin (~30 micron diam), are added 0.01M HCl solution (5 mL at the beginning and add in increments of 1 mL if needed) in suitable container and stirred using magnetic stir bar to form a reasonable slurry suspension for 30 minutes to ensure complete wetting of the resin.
2) Actinium-225 solution (activity = 200 µCi, in 0.01M HCl, approx. volume 60 µl)is added to the flask. Check the pH of the solution (expected to be about pH 2.0) and stirring is resumed for 90 minutes to ensure homogenous distribution of the radioactivity in microspheres.
3) Check for free Ac-225 after centrifugation of the sample by removing a 100 uL aliquot.
4) These labeled microspheres are then filtered using a suitable filtration setup (i.e. 22 micron filter) and washed with 5 ml x 6 times with SWFI. Store each wash separately for alpha counting. Check pH of the last wash solution (expected to be between 6.5-7.0)
5) Transfer the Ac-225 labeled microspheres in a 10 ml vial and resuspend them in sterile pH 7.4 buffer solution (3 mL) to make a suspension. Measure the radioactivity of the microspheres to determine the amount labeled using suitable method. Label the vial as vial no. 1 Ac.

### Part 2) Making Zr-89 labeled microspheres with 2x specific activity

Materials and chemicals:
1) ⁸⁹Zr (20 mCi in pH 8 buffer)
2) Cation exchange resin (~30 micron diam), pH 8 buffer solution (preferably no phosphate), sterile pH 7.4 buffer solution
3) Glassware, magnetic stirrer, labware, sterile water for injection (SWFI)
4) Suitably equipped lab for radiation protection.
5) Dose calibrator and Ge gamma spectroscopy system

Procedure:
1) 0.5 gram of cation exchange resin (~30 micron diam), are added to pH 8 buffer (5 mL at the beginning and add in increments of 1 mL if needed) in suitable container and stirred using magnetic stir bar to form a reasonable slurry suspension for 30 minutes to ensure complete wetting of the resin.
2) Add the Zr-89 solution (20 mCi) in pH 8 buffer to the suspension and continue stirring for 20 minutes.
3) The Zr-89 labeling efficiency is expected to be about 70 % with this procedure.
4) These labeled microspheres are then filtered using a suitable filtration setup (i.e. 22 micron filter) and washed with 5 ml x 6 times with SWFI. Collect all the washes in separate containers to measure labeling efficacy for each isotope.
5) Collect the dual labeled microspheres and resuspend in a 20 ml vial with 5 ml sterile pH 7.4 buffer solution. Label the vial as vial no. 2. Cap the vial.

### Part 3) Preparing the mixture

Using a suitable syringe setup, remove all the contents of the vial no. 1 Ac and transfer them to vial no. 2. Rinse the vial no. 1 Ac with 1 ml sterile pH 7.4 buffer solution, two times, to ensure transfer all of the Ac-225 labeled microspheres to vial no. 2.

Once the mixing is complete, attach the final product vial label to the vial 2 after recording the Ac-225 and Zr-89 activity. This final product vial can be used to make 2 doses of (Ac-225+Zr-89 mixed microspheres) after re-suspending the spheres just before dose drawing.

Although the above description described the core concepts of the radiomicrosphere 20 in the preferred embodiments, many modifications can be made to the above described device to add additional functionality or simply perform the described method using alternative steps. As mentioned, as other isotopes are approved for human use, the isotopes may be substituted for the isotopes in the preferred embodiments. In addition, in alternative embodiments, other known fabrication techniques may be employed to manufacture the radiomicrosphere 20. The radiomicrosphere 20 may be used to treat other type of cancers besides HCC. In alternative embodiments, the radiomicrosphere 20 may be modified to have Beta emitter combined with a dosimetric isotope.

## Claims

1. A radiomicrosphere for use in the treatment of cancer with radioembolization, the radiomicrosphere comprising:
a radiomicrosphere formed from a resin;
at least one isotope attached to the resin for tumoricidal effect which emits primarily alpha particles as the at least one isotope decays;
**characterized in that** daughter radionuclides of the at least one isotope decay are captured by the resin,
wherein the radiomicrosphere comprises a cation exchange resin, wherein the resin is polyfunctional and has at least three different types of functional groups for cation binding, and wherein the at least three functional groups include a carboxylic acid group, a diphosphonic acid group, and a sulfonic acid group.

2. The radiomicrosphere for use according to claim 1, wherein the at least one isotope is Actinium-225 (²²⁵Ac).

3. The radiomicrosphere for use according to claim 1 or 2, further comprising a second isotope attached to the radiomicrosphere used for post-procedure dosimetry.

4. The radiomicrosphere for use according to claim 3, wherein the second isotope is a positron emitter for PET dosimetry.

5. The radiomicrosphere for use according to claim 3 or 4, wherein the second isotope is Zirconium-89 (⁸⁹Zr).

6. The radiomicrosphere for use according to any of claims 3-5, wherein an amount of radiation dose absorbed to both tumor cells and normal liver cells after radioembolization can be determined from the radiomicrosphere within 5 minutes of the start of the PET dosimetry scan.

7. The radiomicrosphere for use according to any of claims 3-6, wherein each radiation treatment contains a total number of around 37 million radiomicrospheres.

8. The radiomicrosphere for use according to any of claims 1-7, wherein the radiomicrosphere consists of a cation exchange resin with at least one radionuclide therein.

9. The radiomicrosphere for use according to any of claims 1-8, wherein the daughter radionuclides of the at least one isotope decay are captured at high efficiency near 100%.

10. The radiomicrosphere for use according to any of claims 1-9, wherein the radiomicrosphere is implanted in a targeted treatment area.

## Patentansprüche

1. Ein Radiomikrokügelchen zur Verwendung bei der Behandlung von Krebs mit Radioembolisation, wobei das Radiomikrokügelchen Folgendes beinhaltet:
ein aus einem Harz gebildetes Radiomikrokügelchen;
mindestens ein an dem Harz befestigtes Isotop für die tumorabtötende Wirkung, das vorwiegend Alphapartikel abgibt, während das mindestens eine Isotop zerfällt;
**dadurch gekennzeichnet, dass** Tochter-Radionuklide des mindestens einen Isotopenzerfalls durch das Harz eingefangen werden,
wobei das Radiomikrokügelchen ein Kationenaustauschharz beinhaltet, wobei das Harz polyfunktional ist und mindestens drei verschiedene Typen von funktionellen Gruppen für die Kationenbindung aufweist, und wobei die mindestens drei funktionellen Gruppen eine Carbonsäuregruppe, eine Diphosphonsäuregruppe und eine Sulfonsäuregruppe umfassen.

2. Radiomikrokügelchen zur Verwendung gemäß Anspruch 1, wobei das mindestens eine Isotop Actinium-225 (²²⁵Ac) ist.

3. Radiomikrokügelchen zur Verwendung gemäß Anspruch 1 oder 2, die ferner ein zweites an dem Radiomikrokügelchen befestigtes Isotop beinhaltet, das zur Dosimetrie im Anschluss an den Eingriff verwendet wird.

4. Radiomikrokügelchen zur Verwendung gemäß Anspruch 3, wobei das zweite Isotop ein Positronenstrahler für die PET-Dosimetrie ist.

5. Radiomikrokügelchen zur Verwendung gemäß Anspruch 3 oder 4, wobei das zweite Isotop Zirconium-89 (⁸⁹Zr) ist.

6. Radiomikrokügelchen zur Verwendung nach einem der Ansprüche 3-5, wobei eine sowohl von Tumorzellen als auch normalen Leberzellen aufgenommene Menge der Strahlendosis nach der Radioembolisation innerhalb von 5 Minuten nach Beginn des PET-Dosimetrie-Scans aus dem Radiomikrokügelchen bestimmt werden kann.

7. Radiomikrokügelchen zur Verwendung nach einem der Ansprüche 3-6, wobei jede Strahlenbehandlung eine Gesamtzahl von etwa 37 Millionen Radiomikrokügelchen enthält.

8. Radiomikrokügelchen zur Verwendung nach einem der Ansprüche 1-7, wobei das Radiomikrokügelchen aus einem Kationenaustauschharz mit mindestens einem darin befindlichen Radionuklid besteht.

9. Radiomikrokügelchen zur Verwendung nach einem der Ansprüche 1-8, wobei die Tochter-Radionuklide des mindestens einen Isotopenzerfalls mit einem hohen Wirkungsgrad von nahezu 100% eingefangen werden.

10. Radiomikrokügelchen zur Verwendung nach einem der Ansprüche 1-9, wobei das Radiomikrokügelchen in einem Zielbehandlungsbereich implantiert wird.

## Revendications

1. Radiomicrosphère destinée à être utilisée dans le traitement du cancer avec une radioembolisation, la radiomicrosphère comprenant :
une radiomicrosphère formée à partir d'une résine ;
au moins un isotope attaché à la résine pour un effet tumoricide, lequel émet principalement des particules alpha alors que le au moins un isotope se désintègre ;
**caractérisé en ce que** les radionucléides fils du au moins un isotope en désintégration sont captés par la résine,
dans laquelle la radiomicrosphère comprend une résine échangeuse de cations, dans laquelle la résine est polyfonctionnelle et a au moins trois types différents de groupes fonctionnels pour la liaison des cations, et dans laquelle les au moins trois groupes fonctionnels comprennent un groupe acide carboxylique, un groupe acide diphosphonique et un groupe acide sulfonique.

2. Radiomicrosphère destinée à être utilisée selon la revendication 1, dans laquelle le au moins un isotope est l'actinium-225 (²²⁵Ac).

3. Radiomicrosphère destinée à être utilisée selon la revendication 1 ou 2, comprenant en outre un second isotope attaché à la radiomicrosphère utilisé pour une dosimétrie post-procédure.

4. Radiomicrosphère destinée à être utilisée selon la revendication 3, dans laquelle le second isotope est un émetteur de positrons pour la dosimétrie par TEP.

5. Radiomicrosphère destinée à être utilisée selon la revendication 3 ou 4, dans laquelle le second isotope est le zirconium-89 (⁸⁹Zr).

6. Radiomicrosphère destinée à être utilisée selon l'une quelconque des revendications 3 à 5, dans laquelle une quantité de dose de rayonnement absorbée à la fois par les cellules tumorales et les cellules hépatiques normales après radioembolisation peut être déterminée à partir de la radiomicrosphère dans les 5 minutes suivant le début du balayage dosimétrique par TEP.

7. Radiomicrosphère destinée à être utilisée selon l'une quelconque des revendications 3 à 6, dans laquelle chaque traitement par rayonnement contient un nombre total d'environ 37 millions radiomicrosphères.

8. Radiomicrosphère destinée à être utilisée selon l'une quelconque des revendications 1 à 7, dans laquelle la radiomicrosphère est constituée d'une résine échangeuse de cations contenant au moins un radionucléide.

9. Radiomicrosphère destinée à être utilisée selon l'une quelconque des revendications 1 à 8, dans laquelle les radionucléides fils d'au moins un isotope en désintégration sont captés avec une efficacité élevée de près de 100 %.

10. Radiomicrosphère destinée à être utilisée selon l'une quelconque des revendications 1 à 9, dans laquelle la radiomicrosphère est implantée dans une zone de traitement ciblée.
